# EUROPÄISCHE PATENTSCHRIFT

(11) **EP 3 029 019 B1**
(45) Veröffentlichungstag und Bekanntmachung des Hinweises auf die Patenterteilung: **04.10.2017**
(21) Anmeldenummer: 14196643.2
(22) Anmeldetag: 05.12.2014
(51) Int. Cl.: C07C 11/04, C07C 2/82, C07C 7/11, F25J 3/02

(54) **Verfahren zur Herstellung von Kohlenwasserstoffen**
Method for the production of hydrocarbons
Procédé de fabrication d'hydrocarbures

(43) Veröffentlichungstag der Anmeldung: 08.06.2016
(73) Patentinhaber: Linde Aktiengesellschaft, 80331 München (DE)
(72) Erfinder: Fritz, Helmut, 81375 München (DE)
(74) Vertreter: Frommberger, Moritz

(56) Entgegenhaltungen:
- WO-A1-99/61852
- WO-A1-2014/011646
- WO-A2-2013/106771

## Beschreibung

Die Erfindung betrifft ein Verfahren zur Herstellung von Kohlenwasserstoffen durch oxidative Methankopplung gemäß dem Oberbegriff des Patentanspruchs 1.

### Stand der Technik

Verfahren zur Herstellung höherer Kohlenwasserstoffe aus Methan durch oxidative Methankopplung (engl. Oxidative Coupling of Methane, OCM) befinden sich derzeit in intensiver Entwicklung. Bei der oxidativen Methankopplung werden ein methanreicher und ein sauerstoffreicher Strom in einen Reaktor eingespeist, wo der Sauerstoff des sauerstoffreichen Stroms und ein Teil des Methans des methanreichen Stroms unter Bildung von Wasser und Nebenprodukten zu höheren Kohlenwasserstoffen, insbesondere dem typischen Zielprodukt Ethylen, reagieren.

Den Reaktor verlässt ein Produktstrom, der aufgrund der derzeit noch geringen Ausbeuten verhältnismäßig große Anteile (über 60%) nicht umgesetztes Methan und vergleichsweise geringe Anteile (unter 10%) Kohlenwasserstoffe mit zwei und mehr Kohlenstoffatomen enthält. In einem entsprechenden Produktstrom sind typischerweise auch 10 bis 20% anderer Komponenten wie Stickstoff, Argon, Wasserstoff, Kohlenmonoxid und/oder Kohlendioxid enthalten. Die Erfindung eignet sich jedoch auch für den Einsatz mit Produktströmen mit höheren Gehalten an Kohlenwasserstoffen mit zwei und mehr Kohlenstoffatomen.

Ein entsprechender Produktstrom muss daher, wie Produktströme aus anderen Verfahren zur Herstellung von Kohlenwasserstoffen grundsätzlich auch, zumindest teilweise in die enthaltenen Komponenten aufgetrennt werden. Dies kann mittels unterschiedlich ausgestalteter Trennsequenzen erfolgen, denen ein entsprechender Produktstrom nach einer geeigneten Aufbereitung, beispielsweise der Abtrennung von Wasser und/oder Kohlendioxid und einer Verdichtung, unterworfen wird.

Trennsequenzen sind für Produktströme aus Verfahren zum Dampfspalten (engl. Steam Cracking) z.B. im Artikel "Ethylene" in Ullmann's Encyclopedia of Industrial Chemistry, Onlineausgabe, 15. April 2007, DOI 10.1002/14356007.a10_045.pub2, beschrieben. Die hierbei eingesetzten Trennschritte können zum Teil auch in den Trennsequenzen für die oxidative Methankopplung eingesetzt werden, wobei hier das Hauptaugenmerk auf der Abtrennung der großen Mengen an Methan sowie der möglichst verlustfreien Gewinnung der Zielprodukte liegt.

Zur Abtrennung von Methan können sogenannte Demethanizer zum Einsatz kommen, die typischerweise eine Destillationssäule umfassen. Am Kopf der Destillationssäule kann ein flüssiger, methanreicher Strom als Rücklauf aufgegeben werden. In solchen Trennsequenzen können zusätzlich auch Absorptionskolonnen in Form sogenannter C2-Absorber zum Einsatz kommen, die ebenfalls mit einem flüssigen, methanreichen Strom als Rücklauf betrieben werden. Der grundsätzliche Unterschied zwischen Absorptionskolonnen und Destillationssäulen ist unten erläutert.

Für die Trennung von Produktströmen aus Verfahren zur oxidativen Methankopplung kann auf die Verwendung von Destillationssäulen ggf. verzichtet werden, d.h. derartige Verfahren können auch nur mit Absorptionskolonnen umgesetzt werden, für die jedoch ebenfalls, wie erwähnt, ein flüssiger, methanreicher Strom als Rücklauf benötigt wird. Auch an anderer Stelle, beispielsweise in Wärmetauschern, kann ein derartiger Strom als flüssiges Kältemittel mit deutlich unter -100 °C eingesetzt werden.

In den genannten Trennsequenzen wird also ein Produktstrom eines entsprechenden Verfahrens oder zumindest ein aus dem Produktstrom gebildeter Strom einer kryogenen Behandlung (Abkühlung in einem Wärmetauscher, ggf. Trennung in einer Destillationssäule und/oder Absorption in einer Absorptionskolonne) unterworfen, bei der zumindest ein flüssiger, methanreicher Strom zum Einsatz kommt.

Der zumindest eine flüssige, methanreiche Strom wird in Trennsequenzen für Produktströme aus Verfahren zum Dampfspalten aus dem Methan der Produktströme selbst gebildet. Bei Verfahren zur oxidativen Methankopplung ist dies jedoch nicht der Fall, insbesondere wenn die hier wie erwähnt vergleichsweise geringen Mengen an Kohlenwasserstoffen mit zwei und mehr Kohlenstoffatomen aus dem Produktstrom nur mittels einer Absorptionskolonne ausgewaschen werden. Mit anderen Worten wird hier zwar ein flüssiger, methanreicher Strom benötigt, der aber in der vorteilhafterweise verwendeten Trennsequenz nicht gewonnen wird.

In Verfahren zur oxidativen Methankopplung wird das aus dem Produktstrom abgetrennte Methan vorteilhafterweise in den verwendeten Reaktor zurückgeführt, wodurch ein Methankreislauf realisiert wird, dem Methan nur durch die Umsetzung im Reaktor und ggf. durch Verluste in der Trennung entzogen wird.

Das entzogene Methan wird durch eine Frischeinspeisung (sogenannter Makeup) ausgeglichen. Hierzu wird beispielsweise gemäß der WO 2014/011646 A1 ein methanhaltiges Einsatzgas der Einlassseite eines entsprechenden Reaktors zugeführt. Um den Eintrag störender Verunreinigungen in den Reaktor zu vermeiden, ist hier aber ein hoher Reinigungsaufwand erforderlich.

Die vorliegende Erfindung stellt sich vor diesem Hintergrund die Aufgabe, Verfahren zur oxidativen Methankopplung entsprechend zu verbessern.

### Offenbarung der Erfindung

Diese Aufgabe wird durch ein Verfahren und eine Anlage zur Herstellung von Kohlenwasserstoffen durch oxidative Methankopplung mit den Merkmalen der unabhängigen Patentansprüche gelöst. Bevorzugte Ausgestaltungen sind Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Flüssige und gasförmige Ströme können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ist hier von "flüssigem Methan" die Rede, sei darunter ein flüssiger Strom verstanden, der reich an Methan ist, jedoch nicht ausschließlich aus Methan bestehen muss.

Gängige Verfahren zur Trennung von Produktströmen aus Verfahren zur Herstellung von Kohlenwasserstoffen umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

In den genannten Verfahren können insbesondere die bereits erwähnten Destillationssäulen und Absorptionskolonnen zum Einsatz kommen. Zur Auslegung und Ausgestaltung entsprechender Vorrichtungen sei auf einschlägige Lehrbücher verwiesen (siehe beispielsweise K. Sattler: Thermische Trennverfahren. Grundlagen, Auslegung, Apparate. Weinheim: Wiley-VCH, 3. Auflage 2001). Destillationssäulen und Absorptionskolonnen werden nachfolgend auch unter dem Begriff "Trennsäulen" zusammengefasst. Eine im Rahmen der vorliegenden Erfindung verwendete Trennsäule wird bei tiefkalten Temperaturen betrieben und ist zur Tieftemperatur-Gaszerlegung eingerichtet. Einer Trennsäule ist typischerweise immer zumindest eine flüssige Fraktion ("Sumpfprodukt") und eine gasförmige Fraktion ("Kopfprodukt") in einem oberen ("Kopf") bzw. unteren Bereich ("Sumpf") entnehmbar.

Bei einer "Destillationssäule" handelt es sich im hier verwendeten Sprachgebrauch um eine Trennsäule, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Fluid) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert oder reich bzw. arm im oben erläuterten Sinne sind.

Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass das Sumpfprodukt mittels eines Sumpfverdampfers erwärmt wird, so dass kontinuierlich ein Teil verdampft und in der Destillationssäule gasförmig aufsteigt. Eine Destillationssäule ist ferner typischerweise mit einem sogenannten Kopfkondensator versehen, in den zumindest ein Teil des Kopfprodukts zu einem Kondensat verflüssigt und als flüssiger Rücklauf am Kopf der Destillationssäule aufgegeben wird. Ein Teil des aus dem Kopfgas erhaltenen Kopfprodukts kann anderweitig, beispielsweise als Produkt, verwendet werden.

Im Gegensatz zu einer Destillationssäule verfügt eine "Absorptionskolonne" typischerweise nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. Die Gasphase wird mit der Lösungsphase "gewaschen". In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen.

### Vorteile der Erfindung

Ein wesentlicher Aspekt der vorliegenden Erfindung ist es, den flüssigen, methanreichen Strom, der für die kryogene Behandlung des Produktstroms aus einem Verfahren zur oxidativen Methankopplung benötigt wird, nicht aus dem Produktstrom selbst zu bilden, sondern diesen extern zuzuführen und diesen gleichzeitig als Frischeinsatz zu verwenden.

Wie zuvor erläutert, wird in entsprechenden Verfahren typischerweise das in dem Produktstrom enthaltene Methan zumindest teilweise (insbesondere möglichst vollständig) rezykliert, d.h. es wird ein Kreislauf geschaffen, dem nur das jeweils umgesetzte und durch Trennverluste verlorene Methan entzogen wird. Die Erfindung sieht nun vor, zumindest einen Teil des Methans, das dem Kreislauf entzogen wird, mittels des Methans auszugleichen, das auch zur kryogenen Behandlung des Produktstroms von extern bereitgestellt wird.

Die Erfindung schlägt hierzu ein Verfahren zur Herstellung von Kohlenwasserstoffen vor, bei dem in einer Reaktionseinheit, die zur Durchführung eines Verfahrens zur oxidativen Methankopplung eingerichtet ist, aus einem methanreichen Einsatzstrom und aus einem sauerstoffreichen Einsatzstrom ein Kohlenwasserstoffe enthaltender Produktstrom erzeugt wird, wobei der Produktstrom oder zumindest ein aus diesem gebildeter Strom in zumindest einer Trenneinheit unter Verwendung zumindest eines flüssigen, methanreichen Stroms kryogen behandelt wird.

Wie zuvor erläutert, umfasst eine "kryogene Behandlung" beispielsweise eine Abkühlung in einem Wärmetauscher, eine Trennung in einer Destillationssäule und/oder eine Absorption in einer Absorptionskolonne, bei der zumindest ein entsprechender flüssiger, methanreicher Strom zum Einsatz kommt.

Das erfindungsgemäße Verfahren sieht auch vor, dass in der zumindest einen Trenneinheit aus in dem Produktstrom sowie aus in dem flüssigen, methanreichen Strom enthaltenem Methan ein Recyclestrom gebildet wird. Dieser Recyclestrom wird als der erwähnte methanreiche Einsatzstrom der Reaktionseinheit zugeführt. Ferner ist erfindungsgemäß vorgesehen, dass der flüssige, methanreiche Strom als Frischeinsatz bereitgestellt wird. Dies bedeutet, dass ein Teil oder sämtliches Methan dieses flüssigen, methanreichen Stroms nicht durch Abtrennung von Methan oder einer methanhaltigen Fraktion aus dem Produktstrom gebildet wird, also nicht zuvor in dem Produktstrom enthalten war, sondern aus einer externen Quelle stammt, beispielsweise einer Erdgaspipeline oder einem Tank. Eine Bereitstellung als Frischeinsatz schließt jedoch eine Aufbereitung eines extern bereitgestellten Stroms zur Gewinnung des flüssigen, methanreichen Stroms nicht aus.

Der flüssige, methanreiche Strom wird vorteilhafterweise unter Verwendung eines druckbeaufschlagten, methanhaltigen Gasgemischs erzeugt, welches separat zu dem Produktstrom bereitgestellt wird, beispielsweise unter Verwendung von druckbeaufschlagtem Erdgas.

Für den erwähnten Ausgleich zumindest eines Teils des dem Kreislauf entzogenen Methans ist vorteilhafterweise vorgesehen, dass der methanreiche Strom in einer Menge eingesetzt wird, in der Methan in einer Menge enthalten ist, die zumindest so groß wie die Menge an Methan ist, die in der Reaktionseinheit umgesetzt wird. Vorteilhafterweise kann der methanreiche Strom in einer noch größeren Menge eingesetzt werden, beispielsweise um Trennverluste auszugleichen.

Erfindungsgemäß ist also vorgesehen, frisches Methan nicht oder nicht ausschließlich gasförmig bereitzustellen und in eine entsprechende Reaktionseinheit selbst oder an deren Einlassseite einzuspeisen, wie dies, wie erwähnt, in der WO 2014/011646 A1 der Fall ist. Die Bereitstellung von frischen, also nicht rezykliertem, Methan erfolgt erfindungsgemäß vielmehr zumindest teilweise in flüssiger Form und dieses Methan wird teilweise oder ausschließlich an geeigneter Stelle in der Trenneinheit eingespeist. Durch die Verwendung flüssigen Methans lässt sich der Eintrag störender Verunreinigungen in den Reaktor vermeiden, so dass sich gegenüber der Verwendung gasförmiger Einsatzströme der Reinigungsaufwand reduziert. Mit anderen Worten wird im Rahmen der vorliegenden Erfindung frisches Methan zumindest teilweise flüssig in den kalten Teil einer entsprechenden Anlage, im Stand der Technik hingegen gasförmig in den warmen Teil einer entsprechenden Anlage eingespeist.

Die Erfindung ermöglicht es durch die externe Bereitstellung von flüssigem Methan bzw. eines entsprechenden methanreichen Stroms mit definierter Zusammensetzung die Anlagenkomponenten des eigentlichen Verfahrens zur oxidativen Methankopplung, d.h. eine entsprechende Reaktionseinheit und die zugehörige Trenneinheit, unabhängig von einer Methanversorgung zu konzipieren. Wie erläutert wird frisches Methan herkömmlicherweise in die Reaktionseinheit oder stromauf hiervon eingespeist und zusätzlich ein flüssiger, methanreicher Strom für die Trennung benötigt. Die erfindungsgemäße Einspeisung eines flüssigen, methanreichen Stroms teilweise oder ausschließlich in die Trenneinheit ermöglicht es, hier eine standardisierte und parametrisierbare Schnittstelle zu schaffen, an der durch die Trenneinheit spezifikationsgerechtes Methan (bzw. ein entsprechender methanreicher Strom) entgegengenommen und an die durch eine Methanversorgung spezifikationsgerechtes Methan (bzw. ein entsprechender methanreicher Strom) übergeben wird. Die Anlagenteile arbeiten im Übrigen unabhängig voneinander.

Wird im Rahmen der vorliegenden Erfindung eine Quelle für ein druckbeaufschlagtes, methanhaltiges Gasgemisch eingesetzt, in der dieses bereits auf einen geeigneten Druck verdichtet ist, sind keine zusätzlichen Verdichter mehr erforderlich, um den flüssigen, methanreichen Strom bereitstellen zu können. Allenfalls muss ein entsprechendes Gasgemisch entspannt werden, wodurch die erforderliche Kälte und/oder Wellenleistung erzeugt werden kann. Derartige vorteilhafte Drücke liegen insbesondere in Erdgaspipelines vor. Auch unverdampftes Flüssigerdgas, beispielsweise aus Tankschiffen, kann eingesetzt werden.

Die Erfindung entfaltet besondere Vorteile bei den eingangs erwähnten Destillationssäulen und Absorptionskolonnen. Durch die Verwendung des flüssigen, methanreichen Rücklaufs, der aufgrund seiner Herkunft im Wesentlichen frei von Ethylen ist, ermöglicht die vorliegende Erfindung auch eine vorteilhafte Gewinnung von im Wesentlichen ethylenfreien Fraktionen. Der Verlust an Ethylen wird damit durch den Einsatz der vorliegenden Erfindung minimiert

Für die genannten Zwecke, also ein Abkühlen in zumindest einem mit dem zumindest einen methanreichen Strom als Kältemittel betriebenen Wärmetauscher und/oder ein Trennen in zumindest einer kryogenen Trenneinrichtung (Destillationssäule und/oder Absorptionskolonne), in der der zumindest eine methanreiche Strom als Rücklauf aufgegeben wird, muss Methan verflüssigt sein bzw. werden, weshalb ein entsprechender Mindestdruck erforderlich ist.

Diese Anforderung erfüllt insbesondere druckbeaufschlagtes Erdgas aus einer entsprechenden Pipeline. Auf diese Weise bereitgestelltes Erdgas weist bereits den erforderlichen Druck auf, der es ermöglicht, eine Verflüssigung zu erzielen und damit einen flüssigen Rücklauf oder einen entsprechenden Strom für einen Wärmetauscher bereitzustellen. Beispielsweise liegt Erdgas in entsprechenden Pipelines bei 40 bis 60 bar vor, der Druck liegt also deutlich oberhalb des für die Verflüssigung erforderlichen Mindestdrucks von mindestens 28 bar abs. (bei ca. -97 °C). Zur üblichen Verwendung als Brenngas erfolgt herkömmlicherweise eine Entspannung auf einen Druck von beispielsweise weniger als 9 bar abs. Erfindungsgemäß wird hingegen entsprechend verdichtetes Erdgas auf höhere Drücke, beispielsweise ca. 36 bar abs., entspannt oder auf einem entsprechend hohen Druck eingesetzt.

Vor der Verwendung wird ein entsprechendes druckbeaufschlagtes, methanhaltiges Gasgemisch, beispielsweise Erdgas, vorteilhafterweise von störenden Komponenten befreit, sofern es diesbezüglich nicht bereits die jeweiligen Anforderungen erfüllt. Die Frage, welche Komponenten als "störend" anzusehen sind, richtet sich nach der angestrebten Verwendung, also der kryogenen Behandlung, die mit dem wenigstens einen methanreichen Strom durchgeführt werden soll. Letztlich wird, wie erläutert, im Rahmen der vorliegenden Anmeldung in einem entsprechenden flüssigen, methanreichen Strom enthaltenes Methan in den erläuterten Kreislauf und die Reaktionseinheit überführt. Anders als bei einer reinen Verwendung als Kältemittel ist daher auch zu beachten, dass der flüssige, methanreiche Strom nicht nur frei von Wasser und Kohlendioxid sowie ggf. von korrosiven Bestandteilen ist, sondern dass in diesem auch keine Komponenten enthalten sind, die sich im Kreislauf akkumulieren würden und/oder in der Reaktionseinheit schädlich sein können.

Vorteilhafterweise wird der flüssige, methanreiche Strom zumindest teilweise aus einem flüssigen Strom erzeugt, welcher wiederum unter Verwendung eines geeigneten Destillations- bzw. Rektifikationsverfahrens aus dem druckbeaufschlagten, methanhaltigen Gasgemisch, beispielsweise dem Erdgas, gebildet wird. Der flüssige, methanreiche Strom kann in dieser Weise gezielt von den erwähnten störenden Komponenten befreit werden, wobei sich die Ausgestaltung eines entsprechenden destillativen Verfahrens nach der zu erzielenden Reinheit richten kann. In bestimmten Fällen kann auf ein destillatives Verfahren verzichtet werden, beispielsweise wenn entsprechend reines Methan zur Verfügung steht. Je nach der erforderlichen Aufreinigung ist eine entsprechende Vorbehandlung unter Verwendung von Verdichtern, Pumpen, Adsorbern etc. vorzusehen, ehe ggf. ein Destillations- bzw. Rektifikationsverfahren durchgeführt wird.

Vorteilhafterweise wird das druckbeaufschlagte, methanhaltige Gasgemisch zunächst zumindest teilweise unter Druck von Verunreinigungen befreit. Die Aufreinigung unter Druck ist besonders vorteilhaft, weil entsprechende Reinigungseinrichtungen aufgrund der Verdichtung nur zur Behandlung vergleichsweise geringer Volumenströme ausgebildet sein müssen. Wasser und Kohlendioxid sind dabei auf jeden Fall aus dem methanhaltigen Gasgemisch zu entfernen, um ein Ausfrieren bei und nach der anschließenden Abkühlung zu vermeiden. Die weiteren zu entfernenden Verunreinigungen richten sich nach der späteren Verwendung:

Vorteilhafterweise werden zur Befreiung von entsprechenden Verunreinigungen beispielsweise Schwefelverbindungen, Kohlendioxid und/oder Quecksilber zumindest teilweise adsorptiv aus dem druckbeaufschlagten, methanhaltigen Gasgemisch entfernt. Zur Regeneration der dabei verwendeten Adsorber kann auch ein in einem Destillations- bzw. Rektifikationsverfahren anfallender Strom, beispielsweise ein verflüssigtes Kopfgas (z.B. stickstoffhaltiges Kopfgas aus einer Trennsäule, die zur Bereitstellung des flüssigen, methanreichen Stroms verwendet wird) oder ein wiederverdampftes Sumpfprodukt verwendet werden.

Besonders vorteilhaft ist es, wenn das druckbeaufschlagte, methanhaltige Gasgemisch in dem Destillationsverfahren an Stickstoff, Wasserstoff und/oder Helium abgereichert wird. Die Entfernung derartiger Komponenten ist insbesondere deshalb vorteilhaft, weil diese möglicherweise in die Produkte oder den erläuterten Kreislauf übergehen.

Besonders vorteilhaft ist hierbei die Verwendung einer Trennwandkolonne. In eine derartige Trennwandkolonne kann auf einer Seite der Trennwand ein Methan, Stickstoff, Wasserstoff und/oder Helium sowie höhere Kohlenwasserstoffe enthaltendes Gasgemisch eingespeist werden, auf der anderen Seite der Trennwand wird hingegen ein an höheren Kohlenwasserstoffen sowie an Stickstoff, Wasserstoff und/oder Helium abgereichertes, flüssiges Gasgemisch erhalten.

Die Erfindung offenbart ferner eine Anlage, die zur Durchführung eines Verfahrens eingerichtet ist, wie es zuvor erläutert wurde, und die sämtliche, zur Durchführung eines entsprechenden Verfahrens eingerichtete Mittel aufweist. Insbesondere weist eine derartige Anlage Mittel zur Bereitstellung des flüssigen, methanreichen Stroms und zu dessen Bereitstellung als Frischeinsatz auf. Diese Mittel umfassen insbesondere eine Destillationssäule, beispielsweise eine Trennwandkolonne.

Die Erfindung wird unter Bezugnahme auf die beigefügten Zeichnungen näher erläutert, welche bevorzugte Ausführungsformen der Erfindung zeigen.

### Kurze Beschreibung der Zeichnungen

Figur 1 zeigt eine Anlage zur Herstellung von Kohlenwasserstoffen gemäß einer Ausführungsform der Erfindung in schematischer Darstellung.
Figur 2 zeigt eine Anlage zur Herstellung von Kohlenwasserstoffen gemäß einer Ausführungsform der Erfindung in schematischer Darstellung.
Figur 3 zeigt eine Anlage zur Herstellung von Kohlenwasserstoffen gemäß einer Ausführungsform der Erfindung in schematischer Teildarstellung.
Figur 4 zeigt eine Anlage zur Herstellung von Kohlenwasserstoffen gemäß einer Ausführungsform der Erfindung in schematischer Teildarstellung.

### Ausführliche Beschreibung der Zeichnungen

In den Figuren sind einander entsprechende Elemente mit identischen Bezugszeichen veranschaulicht. Auf eine wiederholte Erläuterung entsprechender Elemente wird der Übersichtlichkeit halber verzichtet.

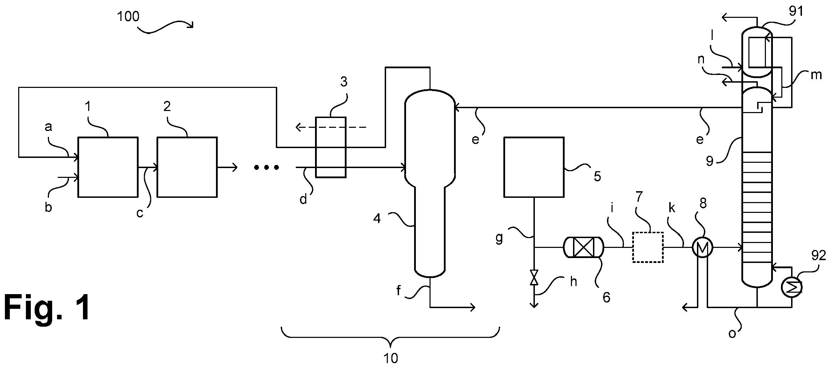

Figur 1 veranschaulicht eine Anlage zur Herstellung von Kohlenwasserstoffen unter Verwendung eines Verfahrens zur oxidativen Methankopplung gemäß einer Ausführungsform der Erfindung. Die Anlage ist insgesamt mit 100 bezeichnet.

Die Anlage 100 umfasst im dargestellten Beispiel eine Reaktionseinheit 1, die zur Durchführung eines Verfahrens zur oxidativen Methankopplung eingerichtet ist, und beispielsweise einen oder mehrere in an sich bekannter Weise ausgebildete beheizte Reaktoren mit geeigneten Katalysatoren umfasst. Die Reaktionseinheit 1 kann auch beispielsweise nachgeordnete Reaktionseinrichtungen umfassen, beispielsweise Reaktionseinrichtungen zum nachgeordneten Dampfspalten.

Der Reaktionseinheit 1 wird ein methanreicher Einsatzstrom a und ein sauerstoffreicher Einsatzstrom b zugeführt und ein Produktstrom c entnommen, der die eingangs erwähnte Zusammensetzung (mehr als 60% Methan, unter 10% Kohlenwasserstoffe mit zwei und mehr Kohlenstoffatomen und 10 bis 20% anderer Komponenten wie Stickstoff, Argon, Wasserstoff, Kohlenmonoxid und/oder Kohlendioxid) aufweisen kann. Dieser wird einem oder mehreren Aufbereitungsschritten, beispielsweise einer Wasserwäsche, einer Aminwäsche, einer adsorptiven Reinigung, einem oder mehreren Trocknungsschritten, einer Verdichtung, einer Abkühlung usw. unterworfen. Entsprechende Schritte sind hier mit Block 2 zusammengefasst. Weitere Schritte, wie hier mit Auslassungspunkten veranschaulicht, können vorgesehen sein.

Ein entsprechend aufbereiteter, insbesondere von Wasser und Kohlendioxid befreiter Strom, nun mit d bezeichnet, wird einer hier mit 10 zusammengefassten Trenneinheit zugeführt und in dieser in einem Wärmetauscher 3 abgekühlt und in eine Absorptionskolonne 4 eingespeist. Auf die Absorptionskolonne 4 wird ein flüssiger, methanreicher Strom e als Rücklauf aufgegeben. Die Absorptionskolonne 4 wird derart betrieben, dass sich in ihrem Sumpf ein überwiegend oder ausschließlich Kohlenwasserstoffe mit zwei und mehr Kohlenstoffatomen enthaltendes Gemisch abscheidet, das als Strom f abgezogen werden kann. Am Kopf der Absorptionskolonne 4 wird ein an Kohlenwasserstoffen mit zwei und mehr Kohlenstoffatomen freies oder nahezu freies Gemisch oder auch reines oder nahezu reines Methan in Form des Stroms a abgezogen.

Anstelle einer einzelnen Absorptionskolonne 4 kann auch jede andere geeignete Einheit verwendet werden, die sich dazu eignet, ein überwiegend oder ausschließlich Kohlenwasserstoffe zwei und mehr Kohlenstoffatome enthaltendes Gemisch zu gewinnen, das als Strom f abgezogen werden kann, und ein an Kohlenwasserstoffen mit zwei und mehr Kohlenstoffatomen freies oder nahezu freies Gemisch bzw. reines oder nahezu reines Methan zu gewinnen, das in Form des Stroms a abgezogen werden kann. Es können beispielsweise kombinierte Einheiten aus einem absorptiv arbeitenden Teil und einem destillativ arbeitenden Teil, ausgebildet als Doppelsäule oder zwei getrennte Säulen, zum Einsatz kommen. Die Auslegung und apparative Ausführung richtet sich nach den Gehalten der einzelnen Komponenten des Stroms d. Ausschlaggebend ist, dass das Gemisch des Stroms a bzw. das reine oder nahezu reine Methan dieses Stroms a frei oder nahezu frei von Kohlenwasserstoffen mit zwei und mehr Kohlenstoffatomen ist. Das Gemisch des Stroms f kann gewisse Mengen an Methan enthalten. Durch den Einsatz der Absorptionskolonne 4 oder einer entsprechenden anderen Einheit müssen die Komponenten, die später im Strom a enthalten sind, nicht kondensiert werden, um einen entsprechenden Strom a zu bilden. Entsprechende Komponenten können daher direkt in die Reaktionseinheit 1 zurückgeführt werden. Die Absorptionskolonne 4 oder eine entsprechende Einheit kann bei Drücken unterhalb von 30 bar, beispielsweise bei 13 bis 17 bar, betrieben werden, allgemeiner bei Drücken, die zu einer Kopftemperatur in der Absorptionskolonne 4 oder einer entsprechenden Einheit von weniger als -97 °C führen. Auf diese Weise enthält der Strom a keine oder nahezu keine Kohlenwasserstoffe mit zwei und mehr Kohlenstoffatomen mehr.

Der Strom e wird gemäß der hier dargestellten Ausführungsform der Erfindung unter Verwendung eines druckbeaufschlagten, methanhaltigen Gasgemischs, hier mit g bezeichnet, gebildet. Der Strom g wird beispielsweise über eine Erdgasversorgung 5, insbesondere eine Pipeline, zur Verfügung gestellt. Das druckbeaufschlagte, methanhaltige Gasgemisch des Stroms g liegt beispielsweise in einer Erdgaspipeline mit einem Druck von 40 bis 60 bar vor und eignet sich damit zur Verflüssigung, ggf. nach vorheriger Entspannung.

Ein Teil des druckbeaufschlagten, methanhaltigen Gasgemischs des Stroms g kann beispielsweise als Strom h über ein nicht bezeichnetes Ventil auf einen Druck von weniger als 9 bar entspannt und anschließend als Brenngas verwendet werden. Der Rest wird einer Aufbereitung zugeführt, die beispielsweise auf einem Druck von 10 bis 50 bar, beispielsweise bei 20 bis 45 bar oder 30 bis 40 bar, arbeitet. Der Druck liegt jedenfalls unterhalb des kritischen Drucks von Methan.

Entsprechende Gasgemische aus Pipelines, wie beispielsweise das druckbeaufschlagte, methanhaltige Gasgemisch des Stroms g, enthalten typischerweise noch Spurenverunreinigungen wie Schwefelverbindungen, Kohlendioxid und Quecksilber. Derartige Verunreinigungen können in einer adsorptiven Reinigungseinrichtung 6 entfernt werden, in der auch beispielsweise die unten erläuterten Ströme n und o zur Regenerierung eingesetzt werden können.

Ein entsprechend aufgereinigter Strom i kann beliebigen weiteren Aufbereitungsschritten 7, beispielsweise einer Kohlendioxidentfernung oder Trocknung, unterworfen werden. Der weiter aufbereitete Strom, nun mit k bezeichnet, wird anschließend in einem Wärmetauscher 8 abgekühlt und in geeigneter Höhe in eine Destillationssäule 9 überführt. Die Destillationssäule 9 dient zur Gewinnung eines methanreichen Kopfstroms aus dem methanhaltigen, druckbeaufschlagten Gasgemisch der Ströme f bzw. k. Liegt der Strom k bereits in ausreichender Reinheit, d.h. insbesondere mit ausreichendem Methangehalt vor, ist auch eine reine Verflüssigung ohne den Einsatz einer Destillationssäule 9 möglich.

Aus der Destillationssäule 9 kann ein gasförmiger Kopfstrom abgezogen, durch den Kondensationsraum eines Kopfkondensators 91, der mit einem geeigneten Kältemittelstrom l betrieben wird, verflüssigt und zumindest zum Teil als Strom m am Kopf der Destillationssäule 9 erneut aufgegeben werden. Nicht im Kopfkondensator 91 der Destillationssäule 9 verflüssigtes Methan kann als Strom n abgezogen und beispielsweise, wie erläutert, für Regenerationszwecke verwendet werden. Der bereits erläuterte Strom e, der im Wesentlichen aus flüssigem Methan besteht, kann am Kopf der Destillationssäule 9 über eine geeignete Flüssigkeitsentnahmeeinrichtung entnommen oder (nicht gezeigt) aus dem Kopfkondensator 91 bzw. einem entsprechenden Behälter abgezogen werden. Es versteht sich, dass die Erfindung unter Verwendung anderer Arten von Kopfkondensatoren, beispielsweise externen Kopfkondensatoren mit separaten Abscheiderbehältern, verwendet werden kann.

Im Sumpf der Destillationssäule 9 scheidet sich eine flüssige Fraktion ab, die überwiegend aus Kohlenwasserstoffen mit zwei und mehr Kohlenstoffatomen bestehen kann. Es ist jedoch auch möglich, die Destillationssäule 9 derart zu betreiben, dass sich in ihrem Sumpf ein Gasgemisch abscheidet, das auch weitere, abzutrennende Komponenten enthält. Auch kann im Sumpf der Destillationssäule 9 noch eine beträchtliche Menge an Methan enthalten sein. Wichtig ist, dass am Kopf der Destillationssäule 9 eine Fraktion gebildet wird, die die oben erläuterte Verwendung zulässt und nur entsprechende Komponenten aufweist.

Ein aus dem Sumpf der Destillationssäule 9 abgezogener Strom o, der nicht in einem Sumpfverdampfer 92 der Destillationssäule 9 verdampft wird, kann ebenfalls als Brenngas verwendet werden, so dass seine Zusammensetzung im Gegensatz zu dem Strom m weniger kritisch ist. Wie dargestellt, kann mit einem aus dem Sumpf der Destillationssäule 9 abgezogenen Strom auch der Wärmetauscher 8 betrieben werden. Auf einen Sumpfverdampfer 92 kann ggf. auch verzichtet werden.

Die Destillationssäule 9 kann bei unterschiedlichen Bedingungen betrieben werden, die auch davon abhängen können, welche spezifische Gaszusammensetzung vorliegt. Beispielsweise können Drücke von 13 bis 36 bar oder 28 bis 36 bar verwendet werden.

Vom Kopf der Absorptionskolonne wird ein Strom a abgezogen, der vorzugsweise den überwiegenden Anteil des in dem Produktstrom c und in dem flüssigen, methanreichen Strom e enthaltenen Methans enthält. Dieser Strom wird als der methanreiche Einsatzstrom a verwendet. Es ist also im dargestellten Beispiel vorgesehen, zur Frischeinspeisung von Methan lediglich den mehrfach erwähnten Strom e zu verwenden. Dieser stammt aus dem separat bereitgestellten, methanhaltigen Gasgemisch des Stroms g, das in geeigneter Weise aufbereitet wird.

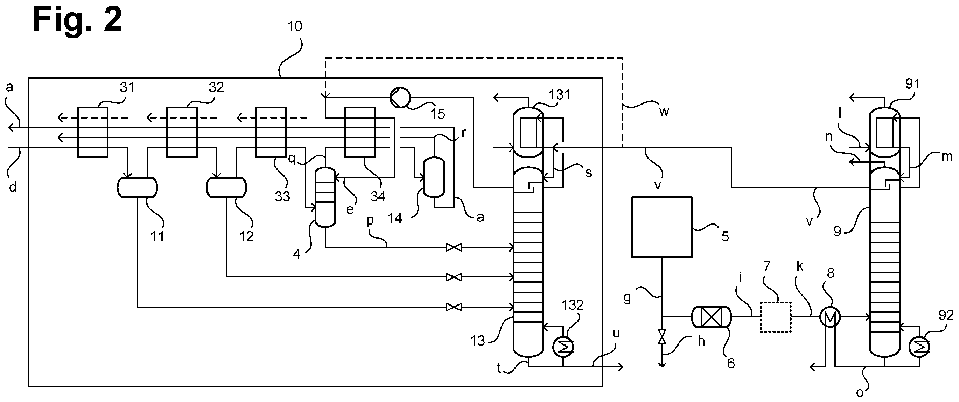

Figur 2 veranschaulicht eine Anlage zur Herstellung von Kohlenwasserstoffen unter Verwendung eines Verfahrens zur oxidativen Methankopplung gemäß einer weiteren Ausführungsform der Erfindung. Auf eine Darstellung der Reaktionseinheit 1 wurde hier verzichtet, es sind lediglich die Figur 1 entsprechenden Ströme a und d gezeigt.

Die Trenneinheit 10 der in Figur 2 gezeigten Anlage entspricht einem Demethzanizer eines Dampfspaltverfahrens. Die Trenneinheit 10 ist stark vereinfacht dargestellt. Auf Die Darstellung einer Vielzahl von Strömen, Ventilen, Wärmetauschern, Behältern usw. wurde verzichtet. Die Trenneinheit umfasst vier (Gegenstrom-)Wärmetauscher 31 bis 34, kann jedoch auch mehr oder weniger entsprechender Wärmetauscher und weitere Wärmetauscher aufweisen. Die Wärmetauscher 31 bis 34 können neben den nachfolgend erläuterten Strömen insbesondere mit geeigneten Kältemittelströmen, hier gestrichelt gezeigt, gekühlt werden.

Der Strom d wird durch den Wärmetauscher 31 geführt, in diesem abgekühlt und anschließend in einen Flüssigkeitsabscheider 11 eingespeist. Hier gasförmig verbleibendes Fluid wird durch den Wärmetauscher 32 geführt, abgekühlt und in einen Flüssigkeitsabscheider 12 eingespeist. Auch hier gasförmig verbleibendes Fluid wird durch den Wärmetauscher 33 geführt, weiter abgekühlt und bei beispielsweise ca. 35 bar abs. und ca. -100 °C in eine Absorptionskolonne 4 überführt.

Am Kopf der Absorptionskolonne 4 wird auch hier ein flüssiger methanreicher Strom e aufgegeben, der in der Absorptionskolonne Kohlenwasserstoffe mit zwei und mehr Kohlenstoffatomen in den Sumpf auswäscht. Im dargestellten Beispiel wird die Absorptionskolonne 4 jedoch derart betrieben, dass sich in ihrem Sumpf ein Gemisch abscheidet, das neben Kohlenwasserstoffen mit zwei und mehr Kohlenstoffatomen noch beträchtliche Mengen Methan enthält. Dieses wird als Strom p abgezogen und in eine Destillationssäule 13 entspannt. In letztere werden in typischen Verfahren zur Aufbereitung von Strömen aus Dampfspaltverfahren auch Kondensate aus den Abscheiderbehältern 11 und 12 entspannt. Dies kann auch in Verfahren zur oxidativen Methankopplung der Fall sein, ist jedoch hier nicht notwendigerweise vorgesehen.

Vom Kopf der Absorptionskolonne 4 wird ein Strom q abgezogen, der im Wesentlichen aus Methan und Wasserstoff besteht. Dieser wird in dem Wärmetauscher 34 auf eine Temperatur unterhalb der Siedetemperatur von Methan bei dem verwendeten Druck abgekühlt und in einen Wasserstoffabscheider 14 überführt, in dem sich im Wesentlichen reines Methan flüssig abscheidet. Erst dieses wird als der methanreiche Einsatzstrom a verwendet, wohingegen Wasserstoff in Form des Stroms r anderweitig eingesetzt wird, beispielsweise für Hydrierungszwecke.

Die Destillationssäule 13 wird derart betrieben, dass sich an ihrem Kopf ein methanreiches Kopfgas, vorzugsweise im Wesentlichen reines Methan, anreichert. Dieses wird abgezogen, durch einen Kondensationsraum eines in seinem Verdampfungsraum mit einem geeigneten Kältemittelstrom betriebenen Kopfkondensators 131 geführt und als flüssiger Rücklauf in Form des Stroms s auf die Destillationssäule 13 aufgegeben. Ein Teil des verflüssigten Stroms s kann abgezogen, mittels einer Pumpe 15 auf den Druck der Absorptionskolonne 4 gebracht und in Form des erwähnten Stroms e als Rücklauf verwendet werden. Es sei ausdrücklich betont, dass die Gewinnung der Ströme s und e auch auf andere Weise, beispielsweise mittels externer Abscheiderbehälter und/oder externer Kopfkondensatoren, erfolgen kann.

Aus dem Sumpf der Destillationssäule 13 kann ein methanarmer Strom t abgezogen werden, der den überwiegenden Anteil der in dem Strom d enthaltenen Kohlenwasserstoffe mit zwei und mehr Kohlenstoffatomen enthält. Ein Teil des Stroms t kann in einem Sumpfverdampfer 132 der Destillationssäule 13 verdampft und erneut in diese eingespeist werden, ein weiterer Teil wird als Strom u entnommen und kann nach beliebigen optionalen Zwischenschritten aus der Anlage ausgeleitet werden.

In der in Figur 2 dargestellten Ausführungsform wird also der Strom e nicht notwendigerweise direkt extern bereitgestellt, er kann auch zunächst zumindest zum Teil aus Methan aus der Destillationssäule 13 bzw. deren Kopfkondensator 131 gebildet werden. Gleichwohl wird auch hier Methan extern bereitgestellt, nämlich in Form des Stroms v. Der Strom v wird wie der zu Figur 1 erläuterte Strom e gewonnen. Auf die obigen Ausführungen wird daher verwiesen. Ein Teilstrom w des Stroms v kann auch für Kühlzwecke eingesetzt werden. Der Strom v kann aber auch insgesamt wie der dargestellte Strom w geführt werden.

Anstelle der einfachen Destillationssäule 9 kann auch eine Trennwandkolonne eingesetzt werden. Die Verwendung einer Trennwandkolonne trägt der Tatsache Rechnung, dass beispielsweise Erdgas, das in Form des druckbeaufschlagten, methanhaltigen Gasgemischs des Stroms g bereitgestellt wird, typischerweise beträchtliche Mengen an Stickstoff enthält. Um zu vermeiden, dass entsprechender Stickstoff in den Strom e bzw. v übergeht, wird in der Trennwandkolonne eine Abreicherung von Stickstoff erzielt. Entsprechender Stickstoff könnte anderenfalls Produkte einer entsprechenden Anlage verunreinigen. Stickstoff könnte auch in den mehrfach erläuterten Kreislauf eingespeist werden und sich in diesem mangels Umsetzung in der Reaktionseinheit 1 im Kreislauf akkumulieren.

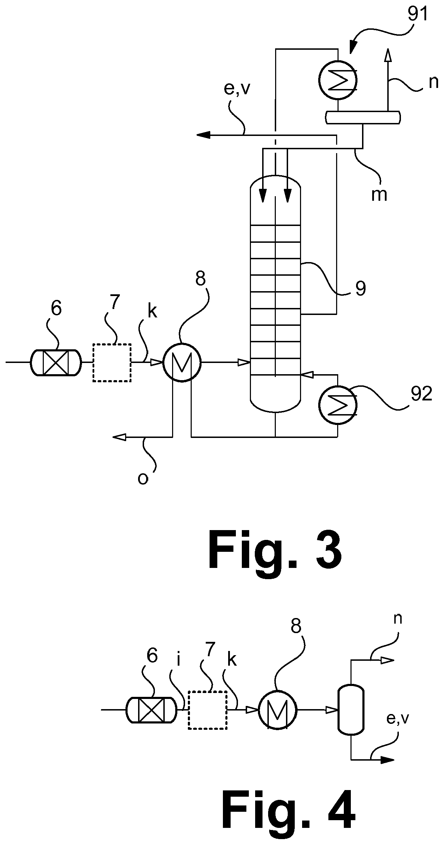

In Figur 3 ist ein Ausschnitt einer erfindungsgemäßen Anlage gezeigt, in der eine entsprechende Trennwandkolonne wie die normale Destillationssäule 9 der Figuren 1 und 2 mit 9 bezeichnet ist. Die Einbindung ergibt sich aus der Bezeichnung der jeweiligen Ströme. Die Trennwandkolonne 9 umfasst im dargestellten Beispiel einen Kopfkondensator 91 und einen Sumpfverdampfer 92. Der noch stickstoffhaltige Gasstrom k wird nach der Abkühlung in dem Wärmetauscher 8 in einen hier links dargestellten Bereich der Trennwandkolonne 9 eingespeist. In einem rechts dargestellten Bereich der Trennwandkolonne 9 kann stickstoffabgereichertes Methan abgezogen und als der Strom e (entsprechend Figur 1) bzw. der Strom v (entsprechen Figur 2) verwendet werden.

Eine besonders einfache Variante ist in Figur 4 veranschaulicht. Hier wird der Strom e bzw. v lediglich durch Verflüssigung eines entsprechend aufgereinigten Stroms k bzw. i erhalten. Diese Variante eignet sich für Fälle, in denen das Gasgemisch des Stroms g bereits eine spezifikationsgerechte Zusammensetzung aufweist. Ist der Strom g bereits frei von anderen störenden Verunreinigungen, kann auch auf die Reinigungseinrichtung 6 verzichtet werden.

## Patentansprüche

1. Verfahren zur Herstellung von Kohlenwasserstoffen, bei dem in einer Reaktionseinheit (1), die zur Durchführung eines Verfahrens zur oxidativen Methankopplung eingerichtet ist, aus einem methanreichen Einsatzstrom (a) und aus einem sauerstoffreichen Einsatzstrom (b) ein Kohlenwasserstoffe enthaltender Produktstrom (c) erzeugt wird, wobei der Produktstrom (c) oder zumindest ein aus diesem gebildeter Strom in zumindest einer Trenneinheit (10) unter Verwendung zumindest eines flüssigen, methanreichen Stroms (e, v) kryogen behandelt wird, **dadurch gekennzeichnet, dass** in der zumindest einen Trenneinheit (10) aus in dem Produktstrom (c) sowie aus in dem zumindest einen flüssigen, methanreichen Strom (e, v) enthaltenem Methan ein Recyclestrom gebildet wird, der als der methanreiche Einsatzstrom (a) der Reaktionseinheit (1) zugeführt wird, und dass der flüssige, methanreiche Strom (e, v) als Frischeinsatz bereitgestellt wird.

2. Verfahren nach Anspruch 1, bei dem der flüssige, methanreiche Strom (e, v) unter Verwendung eines druckbeaufschlagten, methanhaltigen Gasgemischs (g) erzeugt wird, welches separat zu dem Produktstrom (c) bereitgestellt wird.

3. Verfahren nach Anspruch 1 oder 2, bei dem der methanreiche Strom (e, v) in einer Menge eingesetzt wird, die Methan in einer Menge enthält, die zumindest so groß wie die Menge an Methan ist, die in der Reaktionseinheit (1) umgesetzt wird.

4. Verfahren nach einem der vorstehenden Ansprüche, bei dem die kryogene Behandlung ein Abkühlen in zumindest einem mit dem zumindest einen methanreichen Strom (e) als Kältemittel betriebenen Wärmetauscher (34) umfasst.

5. Verfahren nach einem der vorstehenden Ansprüche, bei dem die kryogene Behandlung ein Trennen in einer kryogenen Trenneinrichtung (4, 13) umfasst, in der der zumindest eine methanreiche Strom (e, v) als Rücklauf aufgegeben wird.

6. Verfahren nach Anspruch 5, bei dem eine Absorptionskolonne (4) und/oder eine Destillationssäule (13) als die kryogene Trenneinrichtung verwendet wird.

7. Verfahren nach einem der Ansprüche 2 bis 6, bei dem Erdgas als das druckbeaufschlagte, methanhaltige Gasgemisch (g) verwendet wird.

8. Verfahren nach einem der Ansprüche 2 bis 7, bei dem der methanreiche Strom (e, v) zumindest teilweise aus einem flüssigen Strom erzeugt wird, der unter Verwendung eines Destillationsverfahrens aus dem druckbeaufschlagten, methanhaltigen Gasgemisch (g) gebildet wird.

9. Verfahren nach Anspruch 8, bei dem das druckbeaufschlagte, methanhaltige Gasgemisch (g) vor der Durchführung des Destillationsverfahrens zumindest teilweise von Verunreinigungen befreit wird.

10. Verfahren nach Anspruch 9, bei dem Schwefelverbindungen, Kohlendioxid und/oder Quecksilber zumindest teilweise aus dem druckbeaufschlagten, methanhaltigen Gasgemisch (g) entfernt werden.

11. Verfahren nach einem Ansprüche 8 bis 10, bei dem das druckbeaufschlagte, methanhaltige Gasgemisch (g) in dem Destillationsverfahren an Stickstoff, Wasserstoff und/oder Helium abgereichert wird.

12. Verfahren nach Anspruch 11, bei dem zur Abreicherung des druckbeaufschlagten, methanhaltigen Gasgemischs (g) an Stickstoff, Wasserstoff und/oder Helium in dem Destillationsverfahren eine Trennwandkolonne verwendet wird.

## Claims

1. Process for producing hydrocarbons, where in a reaction unit (1) set up for performing a process for oxidative methane coupling a hydrocarbon-containing product stream (c) is generated from a methane-rich feed stream (a) and from an oxygen-rich feed stream (b), wherein the product stream (c) or at least a stream formed therefrom is cryogenically treated in at least one separation unit (10) using at least one liquid, methane-rich stream (e, v), **characterized in that** in the at least one separation unit (10) from methane present in the product stream (c) and from methane present in the at least one liquid, methane-rich stream (e, v) a recycle stream is formed which is supplied to the reaction unit (1) as the methane-rich feed stream (a), and **in that** the liquid, methane-rich stream (e, v) is provided as a fresh feed.

2. Process according to Claim 1, where the liquid, methane-rich stream (e, v) is generated using a pressurized, methane-containing gas mixture (g) which is provided separately to the product stream (c).

3. Process according to Claim 1 or 2, where the methane-rich stream (e, v) is employed in an amount which comprises methane in an amount at least as great as the amount of methane converted in the reaction unit (1).

4. Process according to any of the preceding claims, where the cryogenic treatment comprises a cooling in at least one heat exchanger (34) operated with the at least one methane-rich stream (e) as coolant.

5. Process according to any of the preceding claims, where the cryogenic treatment comprises a separation in a cryogenic separation device (4, 13) in which the at least one methane-rich stream (e, v) is applied as reflux.

6. Process according to Claim 5, where an absorption column (4) and/or a distillation column (13) are used as the cryogenic separation device.

7. Process according to any of Claims 2 to 6, where natural gas is used as the pressurized, methane-containing gas mixture (g).

8. Process according to any of Claims 2 to 7, where the methane-rich stream (e, v) is generated at least partly from a liquid stream formed using a distillation process from the pressurized, methane-containing gas mixture (g).

9. Process according to Claim 8, where the pressurized, methane-containing gas mixture (g) is at least partly freed of impurities before performance of the distillation process.

10. Process according to Claim 9, where sulfur compounds, carbon dioxide and/or mercury are at least partly removed from the pressurized, methane-containing gas mixture (g).

11. Process according to any of Claims 8 to 10, where the pressurized, methane-containing gas mixture (g) is depleted of nitrogen, hydrogen and/or helium in the distillation process.

12. Process according to Claim 11, where a dividing wall column is used for depleting the pressurized, methane-containing gas mixture (g) of nitrogen, hydrogen and/or helium in the distillation process.

## Revendications

1. Procédé de fabrication d'hydrocarbures, selon lequel un courant de produits (c) contenant des hydrocarbures est généré dans une unité de réaction (1), qui est conçue pour la réalisation d'un procédé de couplage oxydatif de méthane, à partir d'un courant de charge riche en méthane (a) et d'un courant de charge riche en oxygène (b), le courant de produits (c) ou au moins un courant formé à partir de celui-ci étant soumis à un traitement cryogénique dans au moins une unité de séparation (10) en utilisant au moins un courant liquide riche en méthane (e, v), **caractérisé en ce qu'**un courant de recyclage est formé dans ladite au moins une unité de séparation (10) à partir du méthane contenu dans le courant de produits (c) et dans ledit au moins un courant liquide riche en méthane (e, v), qui est introduit dans l'unité de réaction (1) en tant que courant de charge riche en méthane (a), et **en ce que** le courant liquide riche en méthane (e, v) est préparé sous la forme d'une charge fraîche.

2. Procédé selon la revendication 1, selon lequel le courant liquide riche en méthane (e, v) est formé en utilisant un mélange gazeux contenant du méthane sous pression (g), qui est préparé séparément du courant de produits (c).

3. Procédé selon la revendication 1 ou 2, selon lequel le courant riche en méthane (e, v) est utilisé en une quantité qui contient du méthane en une quantité qui est au moins égale à la quantité de méthane qui est mise en réaction dans l'unité de réaction (1).

4. Procédé selon l'une quelconque des revendications précédentes, selon lequel le traitement cryogénique comprend un refroidissement dans au moins un échangeur de chaleur (34) exploité avec ledit au moins un courant riche en méthane (e) en tant qu'agent réfrigérant.

5. Procédé selon l'une quelconque des revendications précédentes, selon lequel le traitement cryogénique comprend une séparation dans un dispositif de séparation cryogénique (4, 13), dans lequel ledit au moins un courant riche en méthane (e, v) est introduit en tant que reflux.

6. Procédé selon la revendication 5, selon lequel une colonne d'absorption (4) et/ou une colonne de distillation (13) sont utilisées en tant que dispositif de séparation cryogénique.

7. Procédé selon l'une quelconque des revendications 2 à 6, selon lequel du gaz naturel est utilisé en tant que mélange gazeux contenant du méthane sous pression (g).

8. Procédé selon l'une quelconque des revendications 2 à 7, selon lequel le courant riche en méthane (e, v) est généré au moins en partie à partir d'un courant liquide qui est formé en utilisant un procédé de distillation à partir du mélange gazeux contenant du méthane sous pression (g).

9. Procédé selon la revendication 8, selon lequel le mélange gazeux contenant du méthane sous pression (g) est au moins partiellement débarrassé des impuretés avant la réalisation du procédé de distillation.

10. Procédé selon la revendication 9, selon lequel les composés de soufre, le dioxyde de carbone et/ou le mercure sont au moins partiellement éliminés du mélange gazeux contenant du méthane sous pression (g).

11. Procédé selon l'une quelconque des revendications 8 à 10, selon lequel le mélange gazeux contenant du méthane sous pression (g) est appauvri en azote, hydrogène et/ou hélium dans le procédé de distillation.

12. Procédé selon la revendication 11, selon lequel une colonne à paroi de séparation est utilisée pour l'appauvrissement du mélange gazeux contenant du méthane sous pression (g) en azote, hydrogène et/ou hélium dans le procédé de distillation.
